(19) Europäisches Patentamt / European Patent Office / Office européen des brevets

(11) **EP 2 783 716 B1**

(12) **EUROPÄISCHE PATENTSCHRIFT**

(45) Veröffentlichungstag und Bekanntmachung des Hinweises auf die Patenterteilung:
**03.08.2022 Patentblatt 2022/31**

(21) Anmeldenummer: **14160868.7**

(22) Anmeldetag: **20.03.2014**

(51) Internationale Patentklassifikation (IPC):
**A61M 1/16** $^{(2006.01)}$ **A61M 1/36** $^{(2006.01)}$

(52) Gemeinsame Patentklassifikation (CPC):
**A61M 1/1609; A61M 1/1613; A61M 1/3658;**
A61M 2205/18; A61M 2205/3313; A61M 2205/3331

(54) **Verfahren und Vorrichtung zur Erkennung einer Rezirkulation in einem Shunt**

Method and device for detecting recirculation in a shunt

Procédé et dispositif de détection d'une recirculation dans un shunt

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.03.2013 DE 102013103218**

(43) Veröffentlichungstag der Anmeldung:
**01.10.2014 Patentblatt 2014/40**

(73) Patentinhaber: **B. Braun Avitum AG**
**34212 Melsungen (DE)**

(72) Erfinder:
• **Ahrens, Jörn**
 **34225 Baunatal (DE)**
• **Moll, Stefan**
 **22587 Hamburg (DE)**

(74) Vertreter: **Winter, Brandl - Partnerschaft mbB**
**Alois-Steinecker-Straße 22**
**85354 Freising (DE)**

(56) Entgegenhaltungen:
EP-A1- 2 292 283 WO-A1-2014/044365
US-A1- 2007 083 145 US-A1- 2008 097 272
US-A1- 2009 054 822

**Beschreibung**

Die Erfindung betrifft ein Verfahren und eine Vorrichtung zur Erkennung einer Rezirkulation in einem Shunt. Bei einer Dialyse kann es aufgrund einer Veränderung des Zugangs, einer Stenose, einem zu geringen Shuntfluss oder aus anderen Gründen zu einer Rezirkulation im Shunt kommen. Eine solche Rezirkulation verursacht einen direkten Rückfluss von gereinigtem Blut aus dem venösen Zugang wieder direkt in den arteriellen Zugang zum Dialysator. Hierdurch wird die Effektivität der Dialyse deutlich reduziert.

Generell wird angestrebt, dies zu vermeiden. Eine Aufgabe der Erfindung liegt allgemein darin, das Auftreten, oder eine deutliche Änderung, einer Rezirkulation zuverlässig erkennen zu können und gegebenenfalls sicherstellen zu können, dass sich die Dialyseleistung während der gesamten Therapie nicht verringert und somit eine effektive Dialyse gewährleistet werden kann.

Aus der DE 699 16 053 T2 ist eine Dialysemaschine zum Bestimmen des Gehalts von Abfallprodukten in einer Dialyseflüssigkeit bekannt. Die Messung der Konzentration von einer oder mehreren Substanzen wird direkt an der während der Dialysebehandlung aus dem Dialysator austretenden Dialyseflüssigkeit unter Einsatz einer spektralfotometrischen Messzelle vorgenommen. Hierdurch können Abfallprodukte wie etwa Harnstoff quantitativ erfasst werden.

Ferner sind aus der EP 2 292 283 A1 Geräte und Methoden zur Erkennung von klinischen und subklinischen Anomalien während einer Nierenersatzbehandlung durch kontinuierliche Messung des Dialysats einer Nierenersatzbehandlung bekannt, bei welchen die Erkennung der Anomalien erreicht wird, indem die Zeitabhängigkeit eines ersten Satzes von Messungen an ein physiologisches Modell angepasst wird, die physiologisch relevanten Parameter extrahiert und weitere in Echtzeit durchgeführte Messungen mit den entsprechenden Modellvorhersagen verglichen werden.

Außerdem ist aus der US 2007/083145 A1 eine Blutreinigungsvorrichtung bekannt mit einem Blutkreislauf mit arteriellen und venösen Blutkreisläufen, einer Blutpumpe, einer Blutreinigungsvorrichtung, einer Indikatoranbringungsvorrichtung, die einen vorbestimmten Indikator auf das Blut aufbringt, das extrakorporal durch den Blutkreislauf fließt, einer Detektionsvorrichtung, die den von der Indikatoranbringungsvorrichtung aufgebrachten Indikator erfasst, einer arithmetischen Vorrichtung, die auf der Grundlage des von der Erfassungsvorrichtung erfassten Indikators eine Rezirkulationsrate berechnet, und einer Berechnungsvorrichtung, die einen idealen extrakorporal zirkulierenden Blutfluss berechnet, der dazu führt, dass die Rezirkulationsrate nicht mehr als ein vorbestimmter Wert ist, wenn die von der arithmetischen Vorrichtung berechnete Rezirkulationsrate größer als der vorbestimmte Wert ist.

Darüber hinaus sind aus der US 2008/097272 A1 eine Vorrichtung und ein Verfahren zur Erkennung von Komplikationen während einer extrakorporalen Blutbehandlung bekannt. Während der Dialysebehandlung kann die Dialyse bzw. die Clearance zu aufeinanderfolgenden Zeitpunkten kontinuierlich auf der Grundlage einer gemessenen Änderung einer physikalischen oder chemischen Kenngröße der Dialyseflüssigkeit, beispielsweise der Ionenkonzentration der Dialyseflüssigkeit, bestimmt werden. Wird eine signifikante Änderung der Dialyse oder Clearance festgestellt, kann die Bestimmung der Rezirkulation auf der Grundlage einer gemessenen Änderung einer physikalischen oder chemischen Kenngröße des Blutes erfolgen. Tritt sowohl eine signifikante Änderung der ermittelten Dialyse als auch der Rezirkulation auf, kann eine Komplikation hinsichtlich eines schlechten Gefäßzugangs angenommen werden. Alternativ kann eine Komplikation hinsichtlich des Dialysators angenommen werden.

Weiter ist aus der US 2009/054822 A1 ein Blutreinigungsapparat bekannt, der einen spezifischen Peak, der dem Blut zu vermitteln ist, deutlicher machen kann und daher die Blutrückführung zuverlässig und präzise erkennen kann.

Schließlich offenbart die am 27. März 2014 veröffentlichte und die Priorität vom 21. September 2012 beanspruchende und damit nur im Hinblick auf Neuheit relevante WO 2014/044365 A1 eine Vorrichtung und ein Verfahren zum Nachweis der Rezirkulation für eine Vorrichtung zur extrakorporalen Blutbehandlung, und eine Vorrichtung zur extrakorporalen Blutbehandlung mit einer Vorrichtung zum Nachweis der Rezirkulation. Die Erfassung einer Rezirkulation kann beispielsweise durch eine Temperaturkontrolle des Blutes oder mittels Ultraschallmessungen erfolgen. Hierbei können jedoch allenfalls punktuelle Rezirkulationsmessungen durchgeführt werden. Dies bringt den Nachteil mit sich, dass Störungen zwischen den Messintervallen unentdeckt bleiben. Weiterhin kann die Behandlung durch die punktuellen Messungen beeinflusst werden. Auch das Schlauchsystem muss geeignet ausgelegt und an den Sensor angeschlossen werden. Ferner ist geschultes Personal zur Auslösung und Begleitung der Messung erforderlich. Mit der Erfindung wird die Zielsetzung einer Online-Überwachung der Therapie bzw. der Dialyseeffektivität erreicht. Die Messung kann hierbei direkt im Dialysatkreislauf erfolgen. Ferner ist keine eigene, zusätzliche Sensorik erforderlich, sondern es kann eine bereits im Dialysegerät vorhandene Sensorikausstattung verwendet werden.

Mit der Erfindung wird ein Verfahren gemäß Patentanspruch 1 geschaffen. Hierdurch kann z.B. eine Rezirkulation im Shunt durch Überwachung des Signalverlaufs erfasst werden, wozu beispielsweise ein UV (Ultraviolett) - Sensor eingesetzt werden kann.

Weiterhin wird mit der Erfindung eine Vorrichtung gemäß Anspruch 12 bereitgestellt, die gleichfalls die Erkennung einer Rezirkulation im Shunt durch Überwachung des Signalverlaufs erlaubt. Auch hier kann ein UV-Sensor eingesetzt werden.

Bei Erkennung einer solchen Rezirkulation können geeignete Gegenmaßnahmen getroffen werden, sodass sichergestellt werden kann, dass sich die Dialyseleistung während der gesamten Therapie nicht verringert und somit eine effektive Dialyse gewährleistet werden kann. Es erfolgt somit gewissermaßen eine Online-Überwachung (Online-Monitoring) bezüglich des Auftretens einer Rezirkulation.

Vorteilhafte Ausgestaltungen der Erfindung sind in den weiteren Ansprüchen angegeben.

Gemäß einem Aspekt der Erfindung wird somit ein Verfahren zum maschinen-seitigen Erfassen einer Rezirkulation von Blut oder einer Änderung einer Rezirkulation während des Betriebs einer Blutbehandlungsmaschine bereitgestellt, wobei während des Betriebs aufeinanderfolgende Messwerte mittels eines Sensors am Abfluss eines Dialysators der Blutbehandlungsmaschine ermittelt werden, und bei dem für einen jeweiligen, vom Sensor erzeugten Messwert ein Grenzwert vorgegeben wird, und bei dem dann, wenn der Messwert den Grenzwert erreicht oder überschreitet, eine Aktion wie etwa die Erzeugung eines optischen und/oder akustischen Warnhinweises eingeleitet wird, wobei ein von dem Sensor ermittelter aktueller Detektorwert in einen Absorbanzwert umgerechnet wird und ein Sollverlauf für die Absorbanz und ebenso für den Verlauf des Detektorsignals berechnet und überwacht wird, um eine plötzliche Rezirkulation bzw. eine plötzliche Rezirkulationsänderung aufgrund einer plötzlichen Veränderung der Absorbanz oder des Verlaufs des Detektorsignals zu erkennen; wobei zur Berechnung des Sollverlaufs der ermittelten Absorbanz und/oder des Sollverlaufs des Detektorsignals mindestens die nachfolgend angegebene Methode eingesetzt wird: der Sollverlauf des Detektorsignals oder der Absorbanz wird durch Linearisierung des Verlaufs des Detektorsignals berechnet, indem zu Beginn des Maschinenbetriebs oder ggf. auch nach einer Blutflussänderung die Steigung des Verlaufs der Detektorsignale ermittelt wird und diese Steigung als Sollwert für den weiteren Verlauf genutzt wird; und wobei die jeweiligen Grenzwerte für die jeweiligen Messwerte gleichartig wie der Sollverlauf berechnet werden, indem zu dem jeweiligen oder einem früheren oder einem erwarteten Messwert ein prozentualer oder fester Wert hinzuaddiert wird, und/oder der Grenzwert prozentual vom Sollwert zu jedem Zeitpunkt berechnet wird, und/oder der Grenzwert mittels eines Versatzwerts (Offset) bezogen auf den Sollverlauf zu jedem Zeitpunkt berechnet wird, und/oder mit Hilfe einer Prädiktion der Verlauf der theoretischen Messwerte ohne Rezirkulation berechnet wird und hierzu eine maximal zulässige obere Grenze als Grenzwert für die Abweichung ermittelt wird, und/oder auf der Basis des Sollverlaufs der Messwerte der Verlauf der Detektorsignale berechnet wird und auf dieser Basis eine maximal zulässige Abweichung für einen nachfolgenden Messwert als Grenzwert berechnet wird.

Der Grenzwert kann z.B. auf der Basis des aktuellen oder mindestens eines vorhergehenden Messwerts oder eines durch Prädiktion wie beispielsweise durch Extrapolation gewonnenen Vorhersagewerts berechnet werden.

Der Grenzwert kann ferner als prozentualer oder fester Versatz (Offset) gegenüber dem aktuellen oder mindestens einem vorhergehenden Messwert oder dem durch Prädiktion gewonnenen Wert, oder auf der Basis einer erlaubten Rezirkulation berechnet werden. Der Sensor kann z.B. ein im Ultraviolettbereich (UV-Bereich) arbeitender Sensor sein und es kann als Lichtquelle eine Leuchtdiode eingesetzt werden, die ein Signal mit einer Wellenlänge im Ultraviolettbereich, beispielsweise von ca. 280 nm, aussendet, wobei der Sensor einen oder vorzugsweise zwei Photodetektoren aufweisen kann.

Vor oder zu Beginn einer Behandlung oder Messung, oder auch während einer Behandlung oder Messung kann ggfls. eine Kalibrierung durchgeführt werden, bei der der Sensor eine definierte Flüssigkeit, vorzugsweise Dialysierflüssigkeit oder Wasser, misst und der hierbei gewonnene Messwert als Nullwert für die nachfolgende Auswertung herangezogen wird.

Der vom Sensor ermittelte aktuelle Detektorwert der Auswerteelektronik wird in einen Absorbanzwert umgerechnet und ein Sollverlauf für die Absorbanz und ebenso für den Verlauf des Detektorsignals wird berechnet und überwacht, um eine plötzliche Rezirkulation bzw. eine plötzliche Rezirkulationsänderung aufgrund einer plötzlichen Veränderung der Absorbanz oder des Verlaufs des Detektorsignals zu erkennen.

Zur Berechnung des Sollverlaufs der ermittelten Absorbanz und/oder des Sollverlaufs des Detektorsignals wird mindestens die nachfolgend angegebene Methode eingesetzt:

der Sollverlauf des Detektorsignals oder der Absorbanz wird durch Linearisierung des Verlaufs des Detektorsignals berechnet, indem zu Beginn einer Therapie oder ggf. auch nach einer Blutflussänderung die Steigung des Verlaufs der Detektorsignale ermittelt wird und diese Steigung als Sollwert für den weiteren Verlauf genutzt wird, und ferner:

der Sollverlauf wird durch Anpassung des exponentiellen Verlaufs der Absorbanz ermittelt, indem zu Beginn der Therapie oder nach einer Blutflussänderung oder während einer laufenden Therapie die Parameter des exponentiellen Verlaufs der Absorbanz ermittelt und als Sollwert für den weiteren Verlauf des Sollverlaufs genutzt werden, und/oder

der Verlauf der Messwerte basierend auf einer Historie der letzten Therapien oder der letzten Steigungen der Messsignale aus vorangegangenen Therapien ermittelt wird und hieraus der Sollverlauf berechnet wird, und/oder der Sollverlauf mit Hilfe der Systemparameter und der Patientendaten in einem Modell berechnet wird.

Die jeweiligen Grenzwerte für die jeweiligen Messwerte werden gleichartig wie der Sollverlauf berechnet, indem zu dem jeweiligen oder einem früheren oder

einem erwarteten Messwert ein prozentualer oder fester Wert hinzuaddiert wird, und/oder
der Grenzwert prozentual vom Sollwert zu jedem Zeitpunkt berechnet wird, und/oder
der Grenzwert mittels eines Versatzwerts (Offset) bezogen auf den Sollverlauf zu jedem Zeitpunkt berechnet wird, und/oder
mit Hilfe einer Prädiktion der Verlauf der theoretischen Messwerte ohne Rezirkulation berechnet wird und hierzu eine maximal zulässige obere Grenze als Grenzwert für die Abweichung ermittelt wird, und/oder
auf der Basis des Sollverlaufs der Messwerte der Verlauf der Detektorsignale berechnet wird und auf dieser Basis eine maximal zulässige Abweichung für einen nachfolgenden Messwert als Grenzwert berechnet wird.

Auf der Basis von mindestens zwei, vorzugsweise drei oder vier Messwerten können z.B. Prädiktionswerte für erwartete zukünftige Messwerte berechnet werden und auf dieser Basis ein jeweils zugehöriger Grenzwert ermittelt werden.

Der Druck am Blut-Einlass des Dialysators und der Druck am Blut-Ausgang des Dialysators können z.B. mittels Drucksensoren überwacht werden, und es kann dann, wenn der Messwert den Grenzwert erreicht oder überschreitet, und die Drücke am blutseitigen Dialysatoreingang und blutseitigen Dialysatorausgang nicht gleichzeitig parallel ansteigen oder die Differenz konstant bleibt, auf Rezirkulation geschlossen werden, während bei Überschreiten oder Erreichen des Grenzwerts und nicht gleichzeitig parallel ansteigenden Drücken bzw. wenn die Differenz zwischen dem Eingang- und Ausgangsdrucksensor wächst, indem das Signal des Drucksensors vor dem Dialysator ansteigt und keine Veränderung des Signals hinter dem Dialysator auftritt, auf Verklottung geschlossen werden kann.

Die bei Erreichen oder Überschreiten des Grenzwerts vorgenommene Aktion kann z.B. in der Erzeugung eines akustischen und/oder optischen Warnsignals bestehen.

Es kann z.B. nicht nur das Auftreten, sondern auch das Ausmaß einer Rezirkulation ermittelt werden, vorzugsweise durch Differenzbildung der Absorbanz zwischen zwei Messpunkten vor und nach der Erkennung einer Rezirkulation mit Hilfe eines Modells, um anhand der Änderung der Absorbanz vom erwarteten Sollwert die Menge bzw. Größe der Rezirkulation zu bestimmen.

Gemäß einem anderen Aspekt der Erfindung wird somit eine Vorrichtung bereitgestellt zum Erfassen einer Rezirkulation von Blut oder einer Änderung einer Rezirkulation während einer laufenden Blutbehandlung mittels eines Dialysators, mit einem am Abfluss des Dialysators angeordneten Sensor zur Ermittlung aufeinanderfolgender Messwerte während der Blutbehandlung, und einer Auswerteeinrichtung, die dazu ausgelegt ist, für einen jeweiligen, vom Sensor erzeugten Messwert einen Grenzwert vorzugeben, und dann, wenn der Messwert den Grenzwert erreicht oder überschreitet, eine Aktion wie etwa die Erzeugung eines optischen und/oder akustischen Warnhinweises einzuleiten, umfassend Mittel zur Umrechnung eines von dem Sensor ermittelten aktuellen Detektorwerts in einen Absorbanzwert und zur Berechnung und Überwachung eines Sollverlaufs für die Absorbanz und ebenso für den Verlauf des Detektorsignals, um eine plötzliche Rezirkulation bzw. eine plötzliche Rezirkulationsänderung aufgrund einer plötzlichen Veränderung der Absorbanz oder des Verlaufs des Detektorsignals zu erkennen; und Mittel zur Berechnung des Sollverlaufs der ermittelten Absorbanz und/oder des Sollverlaufs des Detektorsignals gemäß der nachfolgend angegebenen Methode: der Sollverlauf des Detektorsignals oder der Absorbanz wird durch Linearisierung des Verlaufs des Detektorsignals berechnet, indem zu Beginn des Maschinenbetriebs oder ggf. auch nach einer Blutflussänderung die Steigung des Verlaufs der Detektorsignale ermittelt wird und diese Steigung als Sollwert für den weiteren Verlauf genutzt wird.

Die Vorrichtung kann Mittel zur Ausführung des vorstehend und/oder nachstehend beschriebenen Verfahrens enthalten.

Mit der Erfindung wird auch ein Computerprogrammprodukt bereitgestellt, das Instruktionen zur Steuerung eines Computers zur Ausführung des vorstehend und/oder nachstehend beschriebenen Verfahrens enthält und auf einem Datenträger gespeichert sein kann.

Die Erfindung wird nachstehend anhand von Ausführungsbeispielen unter Bezugnahme auf die Zeichnungen näher beschrieben. Es zeigen:

Fig. 1 ein Blockschaltbild eines Ausführungsbeispiels einer erfindungsgemäßen Vorrichtung,

Fig. 2 ein Flussbild mit Rezirkulation im Shunt während einer Therapie,

Fig. 3 ein Flussdiagramm mit Absorbanz und Rezirkulation auf der Blutseite,

Fig. 4 einen Signalverlauf eines Detektors mit Grenzwerten, und

Fig. 5 einen Signalverlauf des Detektors mit einem Sollwertverlauf und einem Grenzwert.

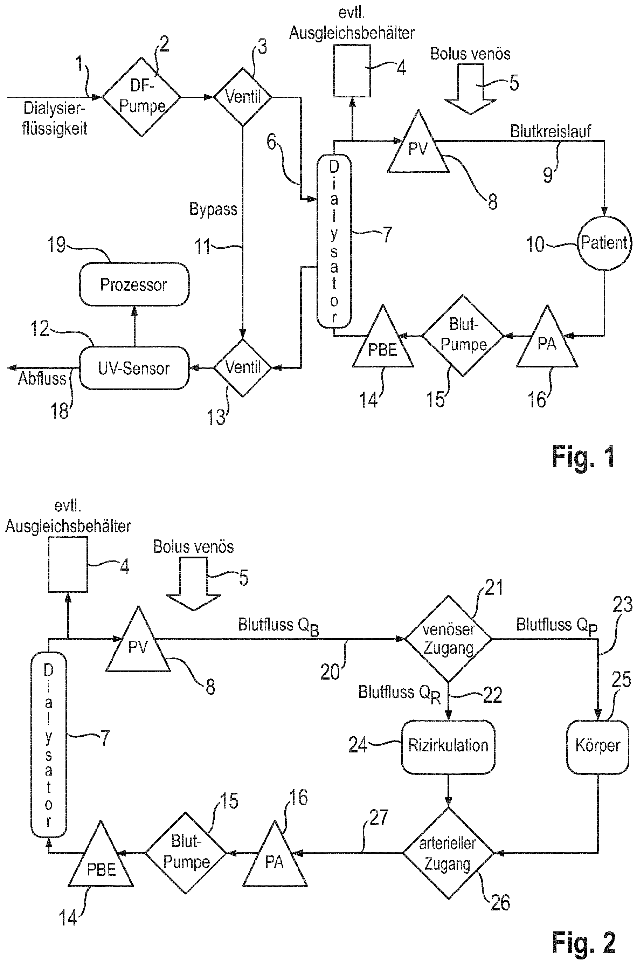

In Fig. 1 ist schematisch ein Ausführungsbeispiel einer erfindungsgemäßen Vorrichtung dargestellt. Einem Dialysator 7 wird Dialysierflüssigkeit 1 über eine Dialysierflüssigkeitspumpe 2 und ein umschaltbares Ventil 3 sowie einen Dialysierflüssigkeitspfad 6 zugeführt. Der Dialysierflüssigkeitspfad 6 durchströmt die Dialysierflüssigkeitsstrecke des Dialysators 7 und strömt ausgangsseitig des Dialysators 7 zu einem Ventil 13. An einem zweiten Eingang des Ventils 13 liegt ein Bypass (Leitung) 11 an, der direkt an einen zweiten Ausgang des Ventils 3 angeschlossen ist. Der aus dem Ventil 13 austretende Fluidstrom wird zu einem Sensor 12, beispielsweise einem UV-Sensor geleitet und strömt dann zu einem Abfluss 18.

Auf der Blutkreislaufseite des Dialysators 7 ist ein Drucksensor 8 für das venöse Blut vorgesehen, der in oder an einem Blutkreislauf 9 vorgesehen ist. Falls erforderlich, kann ein Ausgleichsbehälter 4 für die Aufnahme oder Wiederabgabe von Blut vorgesehen sein, der direkt an dem Blutkreislauf-Ausgang des Dialysators 7 oder auch an einer anderen Stelle angeordnet sein kann. Falls gewünscht oder erforderlich, kann ein schematisch dargestellter Bolus 5 venös in den Blutkreislauf 9 eingespeist werden. Der Dialysepatient ist allgemein mit dem Bezugszeichen 10 bezeichnet.

Auf der arteriellen Seite des Blutkreislaufs ist ein Drucksensor (PA) 16 vorgesehen. Eine Blutpumpe 15 fördert das Blut zur Eingangsseite des Dialysator-Blutstroms, wobei der in diesem Strömungsabschnitt und damit am Dialysatoreintritt herrschende Druck über einen Drucksensor (PBE) 14 detektiert wird.

Im Folgenden wird das UV-Messverfahren näher erläutert. Das Messprinzip des Sensors 12 beruht vorzugsweise auf dem Prinzip der Photometrie. Beispielsweise kann ein Sensor angesetzt werden, wie er in der DE 699 16 053 T2 beschrieben ist. Hierbei besteht eine Lichtquelle aus einer Leuchtdiode LED. Als Detektoren kommen zwei Photodetektoren zum Einsatz. Die Leuchtdiode LED emittiert ein Signal mit einer Wellenlänge von beispielsweise ca. 280 nm. Diese Wellenlänge wird von harnpflichtigen Substanzen absorbiert. Das von der LED ausgesandte Licht tritt durch die Dialysierflüssigkeit hindurch und wird von einem oder beiden Photodetektoren erfasst und die Berechnung der Absorbanz erfolgt auf Basis des entsprechenden elektrischen Signals (Detektorwert).

Der Sensor 12 befindet sich im Abfluss hinter dem Dialysator 7 und misst während der Therapie die Absorbanz A der Dialysierflüssigkeit im Abfluss kontinuierlich. Zu Beginn der Behandlung wird der Nullwert des Detektors ($Detektor_0$) mit reiner Flüssigkeit ermittelt. Als reine Flüssigkeit kann die frisch aufbereitete Dialysierflüssigkeit verwendet werden, die noch keine zu messenden harnpflichtigen Substanzen enthält. Hierfür kann beispielsweise das Ventil 3 auf den Bypass 11 so umgeschaltet werden, dass der Sensor 12 über das gleichfalls entsprechend auf den Bypass 11 umgeschaltete Ventil 13 mit reiner Dialysierflüssigkeit 1 gespeist wird. Als reine Flüssigkeit kann alternativ auch Wasser oder eine andere Substanz verwendet werden, solange sie keine harnpflichtigen Subtanzen enthält.

Der Detektornullwert während der Kalibrierung ist in der nachfolgenden Gleichung mit der Bezeichnung "$Detektor_0$" bezeichnet und zur Berechnung der aktuellen Absorbanz wird dazu der aktuelle "$Detektor_i$" aufgezeichnet.

$$A = \log_{10}\left(\frac{Detektor_0}{Detektor_i}\right)$$

Mit "$Detektor_0$" ist dabei das zu Beginn der Messung und/oder Behandlung kalibrierte Level bzw. Messergebnis bezeichnet, das mit einer Substanz, in der Regel Dialysierflüssigkeit, ohne die zu messenden Substanzen durchgeführt und erhalten wird. Die Kalibrierung wird also vorzugsweise vor dem Anlegen des Patienten bzw. in der Bypass-Umschaltung durchgeführt.

Der Dialysierflüssigkeitsfluss, der Blutfluss und der genutzte Dialysator sind die Haupteinflussfaktoren für die vorherrschende Clearance, die maßgeblich Einfluss auf die Absorbanzmessung im Abfluss hat parallel zur Abhängigkeit des Patienten selber. Abhängig von der Clearance Rate werden die Giftstoffe von der Blutseite über den Dialysator, mittels Konvektion oder Diffusion, auf die Dialysierflüssigkeitsseite überführt und dann verdünnt in der verbrauchten Dialysierflüssigkeit die Absorbanz im Abfluss über den Sensor 12 gemessen. Der Sensor 12 ist im Dialysegerät und die Drucksensoren 8, 14, 16 sind in oder an dem extrakorporalen Schlauchsystem gemäß Fig. 1 in der dargestellten Weise angeordnet.

Im Folgenden wird die Detektion einer Rezirkulation im Shunt beschrieben.

Während einer Rezirkulation im Shunt strömt schon gereinigtes Blut wieder aus dem venösen Zugang zurück in den arteriellen Zugang. Die Rezirkulation in Prozent [%] ist das Verhältnis zwischen gereinigtem Blut $Q_B$ und dem eigentlichen Blutfluss zum Dialysator. Dadurch wird nicht die eigentliche, im Patienten vorhandene Konzentration an Giftstoffen zum Dialysator befördert, sondern lediglich eine Verdünnung mit bereits gereinigtem Blut. Hierdurch verringert sich die Dialyseeffektivität.

In Fig. 2 ist die Blutkreislaufseite des Dialysators 7 detaillierter dargestellt, wobei die bereits in Fig. 1 gezeigten Komponenten mit denselben Bezugszeichen versehen sind und nicht nochmals beschrieben werden.

Der vom Drucksensor 8 gemessene, gereinigte Blutfluss $Q_B$ ist mit dem Bezugszeichen 20 versehen und strömt zu dem venösen Zugang 21, über den der Blutfluss zum Körper 25 über den körpereigenen Blutkreislauf 23 strömt. Bei Auftreten einer Rezirkulation ist dieser mit $Q_P$ bezeichnete Blutfluss zum Körper 25 geringer als der Blutfluss $Q_B$.

Bei Auftreten einer schematisch mit dem Bezugszeichen 24 bezeichneten Rezirkulation strömt Blut über den hier nicht separat dargestellten, aber mit dem Bezugszeichen 22 bezeichneten Shunt als Blutfluss $Q_R$ direkt zum arteriellen Zugang 26 und tritt zu dem, dem Körper 25 zu entnehmenden, zu reinigenden Blut hinzu.

In Fig. 2 ist somit die Rezirkulation im Shunt während einer Therapie veranschaulicht.

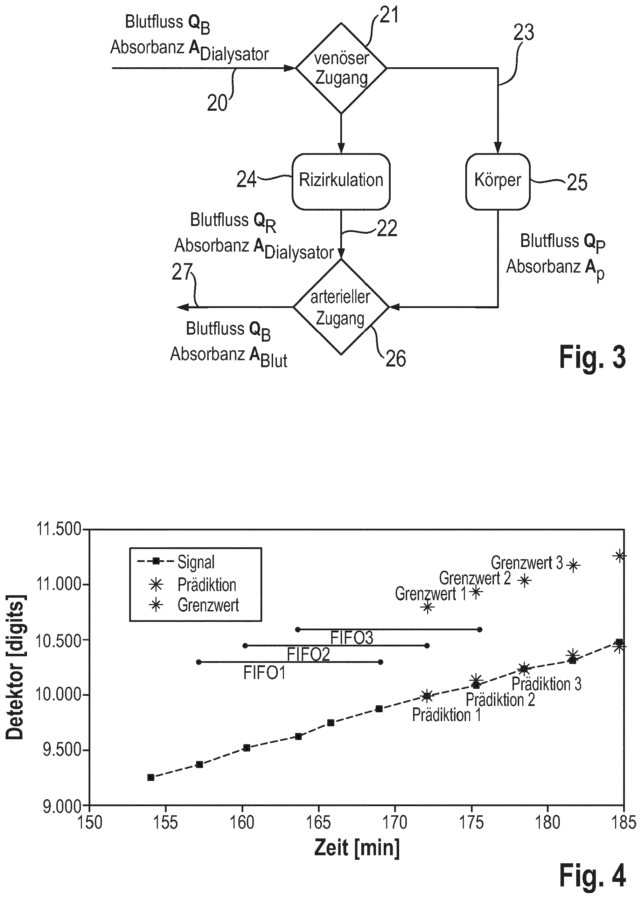

In Fig. 3 ist der in Fig. 2 rechts dargestellte Bereich der Blutströmung mit Rezirkulation in größeren Einzelheiten dargestellt. Hierbei sind nicht nur die Blutflüsse, sondern auch Absorbanzen näher dargestellt.

In der Leitung 20 strömt der Blutfluss $Q_B$, also insgesamt der gereinigte Blutstrom, mit der Absorbanz $A_{Dialysator}$.

In der unerwünschten Rezirkulationsströmung 22 tritt der in der Regel deutlich kleinere Blutfluss $Q_R$ mit einer Absorbanz $A_{Dialysator}$ auf. Der dem körpereigenen Blutkreislauf entnommene, zu reinigende Blutfluss ist mit Qp mit der Absorbanz Ap bezeichnet. Zum arteriellen Zugang 22 fließen somit die beiden Blutströmungskomponenten 22, 23. Im arteriellen Zugang 26 fließt somit ein Blutfluss $Q_B$ mit einer Absorbanz $A_{Blut}$ in einer Leitung 27 zum Drucksensor 16 und weiter zum Dialysator 7. In Fig. 3 ist die Absorbanz und die Rezirkulation auf der Blutseite dargestellt.

Wie aus Fig. 2 und 3 zu erkennen ist, besteht der Blutfluss $Q_B$ zum blutkreislaufseitigen Eingang des Dialysators 7 sowie ebenso auf der blutkreislaufseitigen Austrittsseite des Dialysators 7 auf der Leitung 20 aus der Summe der beiden Anteile von $Q_R$ (Blutfluss bei der Rezirkulation) und $Q_P$ (Blutfluss über den Patienten). Die Rezirkulation R in Prozent ergibt sich somit wie folgt:

$$\text{Rezirkulation R [\%]} = \frac{Q_R}{Q_B}$$

$$Q_B = Q_R + Q_P$$

Im Folgenden wird ein Ausführungsbeispiel einer Methode zur Erkennung und auch quantitativen Messung einer Rezirkulation ohne Eingriff in den körperseitigen Blutkreislauf beschrieben. Mit dieser Messung lässt sich eine plötzliche Rezirkulation bzw. eine plötzliche Veränderung der Rezirkulation durch Überwachung des Signalverlaufs erkennen.

Bei Ausführungsbeispielen des erfindungsgemäßen Verfahrens und der erfindungsgemäßen Vorrichtung kann die Erkennung einer plötzlichen Änderung der Rezirkulation und somit eine plötzliche Veränderung der Dialyseeffektivität während der Therapie durch eine kontinuierliche Messung der Absorbanz bzw. des Detektorsignals im Abfluss erfolgen.

Allgemein ist der Verlauf des Detektorsignals des Sensors 12 während einer Therapie stetig und steigt nahezu linear an, solange im System keine Änderungen wie etwa Änderungen des Blutflusses oder ähnliches auftreten. Der Verlauf der Absorbanz A, die aus dem Detektorsignal berechnet wird, hat hierbei den Verlauf einer einfach bzw. auch doppelt exponentiellen Funktion. Die vom Sensor 12 aufgenommenen Detektorwerte besitzen in ihrem Verlauf eine im Wesentlichen lineare Kennlinie über eine Therapie.

Eine Abweichung von der linearen Steigung bei verschiedenen Patienten im Detektorsignal kann z.B. durch eine abschnittsweise Linearisierung kompensiert werden. Die Berechnung der Absorbanz A aus dem Detektorwert kann mit der folgenden Formel erfolgen:

$$A = \log_{10}\left(\frac{Detektor_0}{Detektor_i}\right)$$

Mit "Detektor$_0$" ist dabei der Messwert des Sensors 12 bezeichnet, bei dem keine Absorbanz von Substanzen existiert, also beispielsweise der zu Beginn der Behandlung bei der Kalibrierung mit Wasser oder Dialysierflüssigkeit erhaltene Wert. Basierend darauf wird dann die Absorbanz zu jedem Zeitpunkt relativ zum Wert "Detektor$_0$" auf Basis des aktuellen Detektormesswerts "Detektor$_i$" berechnet.

Zur Durchführung der Messung, d.h. zur Auswertung der von dem Sensor 12 erzeugten Messsignale ist eine Auswerteeinrichtung 19 (siehe Fig. 1) vorgesehen, die mit dem Sensor 12 über eine Leitung, oder ggf. auch drahtlos, verbunden ist. Die Auswerteeinrichtung 19 kann als Prozessor ausgelegt sein, der die erforderliche Verarbeitung der Messsignale des Sensors 12 durchführt und ggf. auch die Steuerung des Sensors 12, d.h. dessen LED Intensität und dergleichen, steuern kann, um den Sensor im optimalen und stabilen Messbereich zu betreiben. Die Auswerteeinrichtung 19 kann weiterhin mit einem, mehreren oder allen Ventilen 3, 13 für deren Steuerung verbunden sein, und kann ferner optional auch mit einem, mehreren oder allen der Drucksensoren 8, 14 und ggf. 16 verbunden sein, um die von diesen erzeugten Drucksignale zu empfangen und diese Daten bei der Steuerung der Vorrichtung und/oder der Auswertung der Messsignale des Sensors 12 zu berücksichtigen.

Der Sensor 16 kann hierbei zusätzlich, zu dem Drucksensor 14 vor dem Dialysator den Druck vor der Blutpumpe 15 erfassen und bei der Auswertung der Signale des Sensors 12 berücksichtigen.

Wenn während des Verlaufs der Therapie, d.h. der Dialyse, eine plötzliche Rezirkulation im Shunt auftreten sollte, führt dies zu einer rapiden Änderung der Konzentration im Dialysierflüssigkeitsfluss, der vom Sensor 12 überwacht wird. Dies liegt daran, dass der Anteil der dem Blutstrom über den Dialysator 7 entzogenen Giftstoffe bei konstanter Clearance abnimmt, also weniger Giftstoffe aus dem Blutkreislauf entnommen werden. Diese Änderung kann besonders gut detektiert werden, wenn der Blutfluss konstant ist. Demgegenüber haben Änderungen des Dialysierflüssigkeitsflusses nur geringe Auswirkungen auf die Clearance, aber einen Einfluss auf die Absorbanz, da bei unterschiedlichen Flüssen eine unterschiedliche Verdünnung der gleichen Menge auftritt. Änderungen des Dialysierflüssigkeitsflusses können daher beispielsweise durch Normierung der Absorbanz auf einen Dialysierflüssigkeitsfluss erfolgen. Dabei wird der zu Beginn eingestellte Dialysierflüssigkeitsfluss (bspw. 500ml/min) als Normierung genutzt und alle Änderungen danach auf diesen normiert. Damit ist es möglich, bei gleicher Menge von Substanzen im Abfluss die gemessene Absorbanz, proportional zur Konzentration, zu normieren, die auf Grund der unterschiedlichen Verdünnung je nach Dialysierflüssigkeitsfluss ansonsten linear variieren würde. Eine Kompensation der gering variierenden Clearance kann durch eine parallele Messung oder ein Modell korrigiert werden.

Die durch eine plötzliche Rezirkulation oder Rezirkulationsänderung verursachte verringerte Konzentration im Blutkreislauf führt dazu, dass zwar mit der gleichen Clearance-Leistung im Filter gereinigt wird, jedoch effektiv weniger Giftstoffe aus dem Blut entfernt werden, als dies ohne Rezirkulation gegeben wäre. Dies führt zu einem Anstieg des Detektorsignals und zum Abfall der berechneten Absorbanz des Sensors 12, die proportional zur Konzentration ist.

Sofern eine Änderung im System, beispielsweise bei einer Blutflussänderung aufgrund einer veränderten Ansteuerung der Blutpumpe 15 erfolgt, muss die Steigung des Detektorsignals des Sensors 12 relativ zur Konzentration neu bewertet werden. Diese Steigung ist abhängig vom Patientenvolumen und von der Clearancerate, d.h. auch abhängig von Blutfluss und Filter, z.B. im Dialysator 7.

Im Folgenden werden Möglichkeiten zur Ermittlung des Sollverlaufs beschrieben.

Zur Überwachung des Sollverlaufs für die Absorbanz und ebenso des Detektorsignalverlaufs sind verschiedene Möglichkeiten einsetzbar, die alle das Ziel haben, den Sollverlauf der Therapie, d.h. der Blutreinigung, zu ermitteln, um hierbei eine plötzliche Rezirkulation zu erkennen.

Für die Berechnung des Sollverlaufs wird den Verlauf anhand einer Linearisierung des Detektorsignalverlaufs des Sensors 12 ermittelt. Hierzu kann z.B. zu Beginn der Therapie, d.h. der Blutwäsche, oder beispielsweise auch nach einer gezielten Blutflussänderung z.B. durch Ansteuerung der Blutpumpe 15, die Steigung des Detektorsignalverlaufs ermittelt und als Sollverlauf für den weiteren Verlauf des Detektorsignalverlaufs genutzt werden.

Weiterhin ist es zur Berechnung des Sollverlaufs beispielsweise ferner möglich, den Verlauf durch Anpassung (Fittung) des exponentiellen Verlaufs der Absorbanz zu ermitteln, indem zu Beginn der Therapie, d.h. der Blutwäsche, oder ggf. auch nach einer Blutflussänderung, die jeweiligen Parameter, z.B. die Absorbanz im Patient, ermittelt und zur Berechnung des Sollverlaufs genutzt werden.

Zusätzlich ist es zur Berechnung des Sollverlaufs ferner möglich, beispielsweise den Verlauf anhand einer Historie der letzten, ermittelten Steigungen des Absorbanzverlaufs des Patienten zu ermitteln. Hierbei kann z.B. individuell für jeden Patienten, in Kombination mit Geräteparametern wie etwa des Filters und der Flussraten, der Sollverlauf aufgezeichnet oder bestimmt werden. Es ist auch möglich, dies anhand eines mathematischen Models zu ergänzen oder vollständig zu berechnen.

Ferner kann die Bestimmung des Sollverlaufs z.B. mit Hilfe aller Systemparameter und den Patientendaten berechnet werden, wobei hierbei ggf. ein Ein- oder Multi-Kompartiment-Modell berücksichtigt werden muss, mit dem Vorteil, dass die Bestimmung der Absorbanz $A_P$ des dem Patienten über den arteriellen Zugang 26 entnommenen Bluts durch eine Messung, z.B. mit Hilfe des UV-Sensors 12, erfolgt.

Die vorstehend beschriebenen Methoden und Möglichkeiten der Bestimmung des Sollverlaufs der Konzentration können analog oder identisch auch für die Berechnung von nachfolgend näher erläuterten Grenzwerten eingesetzt werden, wobei dies auf den Verlauf der Absorbanz, z.B. ähnlich einer e-Funktion oder auch bezüglich des Verlaufs der Detektorwerte des Sensors 12, beispielsweise nahezu linear, bezogen werden kann.

Im Folgenden wird die Bestimmung eines oder mehrerer Grenzwerte zur Erkennung einer plötzlich auftretenden oder sich plötzlich ändernden Rezirkulation erläutert.

Basierend auf dieser ggf. linearisierten Funktion des Detektorwertverlaufs, also beispielsweise des Sollverlaufs, kann der Grenzwert z.B. prozentual vom Wert des Sollverlaufs zu jedem Zeitpunkt berechnet werden. Dies kann anhand der nachfolgend angegebenen Gleichung erfolgen:

$$\text{Det}_{\text{Grenzwert}} = \text{Det}_{\text{Sollverlauf\_i}} * \text{Grenzwert}_\%$$

Alternativ oder zusätzlich kann der Grenzwert auch mittels eines Offsets, d.h. mittels eines Versatzes oder einer festen Abweichung, bezogen auf den Sollverlauf, zu jedem Zeitpunkt berechnet werden. Hierbei ergibt sich dann die folgende Gleichung, anhand derer der Grenzwert berechnet wird:

$$Det_{Grenzwert} = Det_{Sollverlauf\_i} + Offset$$

Alternativ hierzu kann auch eine Mischform aus den beiden vorgenannten Methoden eingesetzt werden, beispielsweise dadurch, dass dem mit dem prozentualen Grenzwert multiplizierten Dektor-Sollverlauf zum jeweiligen Zeitpunkt der z.B. feste Offsetwert hinzuaddiert wird und hieraus der Grenzwert $Det_{Grenzwert}$ berechnet wird. Hierbei ergibt sich dann beispielsweise die folgende Gleichung:

$$Det_{Grenzwert} = Det_{Sollverlauf\_i} * Grenzwert_{\%} + Offset$$

Die maximale obere Grenze einer Abweichung, bei der noch nicht auf plötzliche Rezirkulation geschlossen wird, kann ebenfalls mit Hilfe einer Prädiktion des ohne Rezirkulation eintretenden Verlaufs der theoretischen Messwerte berechnet werden.

Dieser Grenzwertverlauf entspricht ebenfalls der absoluten Rezirkulation, wenn zu Beginn keine Rezirkulation im Shunt besteht, bzw. dies zu Beginn durch eine Messung bestätigt wird, wie es beispielsweise vorstehend oder nachstehend erläutert ist.

Auf der Basis des Sollwertmessverlaufs erfolgt die Berechnung von $Det_{Sollverlauf\ i+1}$, d.h. des nächsten zu erwartenden Sollverlaufwerts, wobei dieser Wert $Det_{Sollverlauf\ i+1}$ dann zur Berechnung der maximal erlaubten Abweichung für den nächsten Messwert bei einem konstanten System ohne Änderung, d.h. bei normal verlaufender Dialyse ohne Rezirkulation, dient.

In Fig. 4 ist ein Beispiel für einen möglichen Verlauf des Detektorsignals, d.h. des vom Sensor 12 erzeugten Detektorsignals dargestellt. In Fig. 4 sind die tatsächlichen Messsignale mit Rechtecken dargestellt. Grenzwerte und Prädiktionen sind mit Sternen symbolisiert.

Hierbei kann für den in Fig. 4 gezeigten "Grenzwert 1" z.B. eine FIFO (First in - first out)-Verarbeitung, beispielsweise eine gleitende Durchschnittsbildung, von Minute 157 bis 169 gemäß Fig. 4 gebildet werden. Mit Hilfe dieser Punkte wird das erwartete Signal "Prädiktion 1" für die nächste Messung in der Minute 172 berechnet. Aus dem Wert der "Prädiktion 1" wird der Grenzwert 1 berechnet, indem beispielsweise ein bestimmter Wert zu der Prädiktion 1 hinzuaddiert wird. Dieser Grenzwert 1 wird dann später mit der eigentlichen Messung in der Minute 172 verglichen. Sollte dieser bei der Minute 172 ermittelte Messwert oberhalb des Grenzwerts 1 oder direkt auf diesem liegen, kann das System so ausgelegt sein, dass eine Aktion getriggert wie etwa eine Fehlermeldung ausgegeben wird. Zudem oder alternativ kann auch eine Messung gestartet werden, mit der beispielsweise der Grad, d.h. das Ausmaß einer Rezirkulation bestimmt wird.

In Fig. 4 sind die ermittelten Prädiktionen Prädiktion 1, Prädiktion 2, Prädiktion 3, usw. jeweils in Überlagerung mit den tatsächlich gewonnenen Messwerten (Quadrate) dargestellt. Zudem sind die hierbei zugehörigen Grenzwerte Grenzwert 1, Grenzwert 2, Grenzwert 3 mit einem konstanten oder beispielsweise prozentualen Abstand zu den durch die Prädiktion gewonnenen Vorhersagewerten festgelegt. Bei dem Ausführungsbeispiel wird beispielsweise alle drei Minuten eine Messung mittels des Sensors 12 durchgeführt, sodass eine quasi-kontinuierliche Überwachung während der gesamten Dialysebehandlung gewährleistet werden kann und plötzliche Dialysierflüssigkeitsveränderungen als Kennzeichen einer Rezirkulation ausgewertet werden können.

Bei der Abbildung gemäß Fig. 4 ist der Signalverlauf des Detektorsignals des Sensors 12 mit FIFO und jeweiligen Grenzwerten dargestellt. Hierbei ist auf der Abszisse die Zeit in Minuten aufgetragen, während auf der Ordinate das vom Detektor gewonnene Messsignal in Einheiten (Digits) von beispielsweise 9,000 bis 11,500 aufgetragen ist.

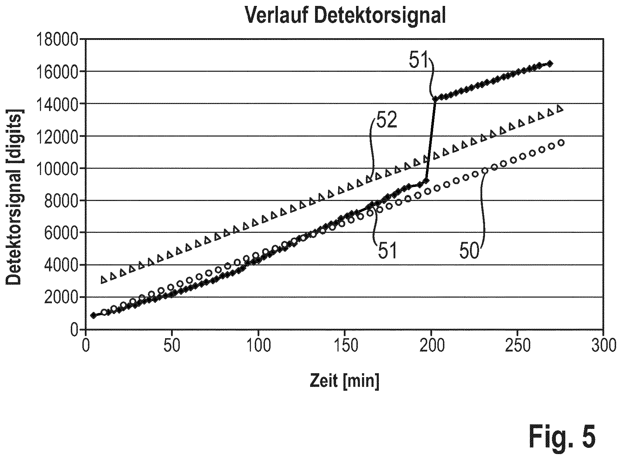

In Fig. 5 ist ein Beispiel eines Signalverlaufs 51 des Detektorsignals des Sensors 12 mit Sollwertverlauf 50 und Grenzwertverlauf 52 dargestellt.

Wie aus Fig. 5 ersichtlich ist, steigt der Sollwertverlauf 50 linear an, ebenso wie der Grenzwertverlauf 52, wobei der Grenzwertverlauf 52 mit gewissem Abstand oberhalb des Sollwertverlaufs 50 in konstantem Abstand parallel verläuft. Das eigentliche Detektorsignal 51 verläuft bis ungefähr zur Minute 200 im Wesentlichen relativ nahe bei dem Sollwertverlauf 50, springt dann aber aufgrund einer plötzlich auftretenden Rezirkulation nach oben und verläuft anschließend deutlich oberhalb des Grenzwertverlaufs 52. Dieses Überschreiten des Grenzwerts gemäß Grenzwertverlauf 52 wird durch die Auswerteeinrichtung 19 (Fig. 1) erkannt und es werden entsprechende Maßnahmen wie etwa die Abgabe einer akustischen und/oder optischen Warnanzeige bzw. eine Umsteuerung des Dialyseverlaufs getroffen. In Fig. 5 ist wiederum auf der Abszisse die Zeit aufgetragen, während auf der Ordinate das vom Sensor 12 abgegebene Detektorsignal in Einheiten (Digits) aufgetragen ist.

Die Berechnung der Grenzwerte kann als prozentualer oder fester Offset (Wert) auf den aktuellen Messwert des Detektorsignalverlaufs 51 bezogen sein, oder kann basierend auf prozentualen Angaben berechnet werden. Der jeweilige Grenzwert kann auch auf der Basis einer als erlaubt angegebenen Rezirkulation berechnet werden oder sein.

Mit den nachstehend angegebenen Gleichungen 3.1 bis 3.5 lässt sich gemäß der nachstehend angegebenen Formel 3.6 die maximal erlaubte Absorbanz, bzw. nach Umwandlung, der maximal erlaubte Detektorwert oder der Grenzwert ermitteln.

Hierbei ist mit dem Buchstaben "A" die Absorbanz bezeichnet, während die Konzentration mit dem Buchstaben "C" bezeichnet ist und ein jeweiliger Berechnungsfaktor mit "F" angegeben ist. Die Absorbanz "A" ist im Wesentlichen proportional oder gleich der mit dem Faktor "F" multiplizierten Konzentration "C".

*Absorbanz* $[A] \sim$ *Faktor* $[F] \cdot$ *Konzentration* $[C]$

$$C_{Dialysat} = \left( \frac{C_{Blut} \cdot Q_{Clearance}}{Q_{Dialysat}} \right) \sim A_{Dialysat} = \left( \frac{A_{Blut} \cdot Q_{Clearance}}{Q_{Dialysat}} \right) \tag{3.1}$$

$$A_{Dialysat} = \left( \frac{\left( A_P(1-R) + A_{Blut} \cdot \left( \frac{Q_{Blut} - Q_{Clearance}}{Q_{Blut}} \right) R \right) \cdot Q_{Clearance}}{Q_{Dialysat}} \right) \tag{3.2}$$

Ohne Rezirkulation gilt:

$$A_{Dialysat+n+1} = \left( Steigung \left( FIFO \Big|_{i=n-4}^{i=n} (Detektor_i) \right) \right) \cdot Zeit(n+1) \tag{3.3}$$

$$A_{Blut\_n+1} = \left( \frac{A_{Dialysat\_n+1} \cdot Q_{Dialysat}}{Q_{Clearance}} \right) \tag{3.4}$$

Unter der Annahme, dass sich die Konzentration im Patienten $A_P = A_{Blut+1}$ auf Grund der Rezirkulation nicht verändert, kann der Grenzwert mit der nachfolgenden Näherung bestimmt werden, und weiter bei Auftreten einer Rezirkulation ebenfalls der Grad der Rezirkulation bestimmt werden.

$$A_{Blut} \cdot Q_B = A_P \cdot Q_P + A_{Dialysator} \cdot Q_R$$

$$A_{Blut} \cdot Q_B = A_P \cdot Q_P + A_{Blut} \cdot \left( \frac{Q_{Blut} - Q_{Clearance}}{Q_{Blut}} \right) \cdot Q_R$$

$$A_{Blut} = A_P(1-R) + A_{Blut} \cdot \left( \frac{Q_{Blut} - Q_{Clearance}}{Q_{Blut}} \right) R$$

$$\tag{3.5}$$

Siehe zu den vorstehenden Gleichungen (3.1) bis (3.5) auch die entsprechenden Eintragungen in den Figuren 1 bis 3. Insbesondere Fig. 3 veranschaulicht den Blutfluss $Q_B$ und die Absorbanz $A_{Dialysator}$ des aus dem Dialysator 7 austretenden gereinigten Bluts 20, während das in den Dialysator 7 einströmende, zu reinigende Blut den Blutfluss $Q_B$ und die Absorbanz $A_{Blut}$ aufweist. Das aus dem Körper entnommene Blut weist den Blutfluss $Q_P$ und die Absorbanz $A_P$ auf.

Der Blutfluss bei einer Rezirkulation ist mit $Q_R$ bezeichnet und weist die Absorbanz $A_{Dialysator}$ auf.

$$A_{Dialysat\_n+1} = \left( \frac{A_{Blut\_n+1} \cdot Q_{Clearance}}{Q_{Dialysat}} \right)$$

$$A_{Blut\_i=} = \left( \frac{A_{Dialysat\_n+1} \cdot Q_{Dialysat}}{Q_{Clearance}} \right) \cdot (1-R) + \left( \frac{A_{Dialysat\_n+1} \cdot Q_{Dialysat}}{Q_{Clearance}} \right) \cdot R$$

$$A_{Blut\_i=} = \left( \frac{A_{Dialysat\_n+1} \cdot Q_{Dialysat}}{Q_{Clearance}} \right) \cdot (1-R) + \left( A_{Blut\_i-1} \right) \cdot R \qquad (3.6)$$

$$A_{Dialysat\_max} = \left( \left\{ \left( \left( \frac{A_{Dialysat+n+1} \cdot Q_{Dialysat}}{Q_{Clearance}} \right) \cdot (1-R) + \left( A_{Blut\_i} \right) \cdot R \right) \right\} \cdot \frac{Q_{Clearance}}{Q_{Dialysat}} \right)$$

Der Wert $A_{Dialysat\_max}$ gibt dabei den Grenzwert an, den das System bei einer Rezirkulation von [%] erreichen würde. Das Detektorsignal wird dann durch die folgende Umrechnung ermittelt.

$$Detektor_{max\_n+1} = \frac{Detektor_0}{10^{A_{Dialysat\_max}}} \qquad (3.7)$$

Das maximale Detektorsignal zum Zeitpunkt n+1 ($Detektor_{max\_n+1}$) ist dabei gleich dem Detektornullwert $Detektor_0$, geteilt durch $10^{A_{Dialysat\_max}}$.

Im Folgenden wird ein Beispiel erläutert. Wenn z.B. eine Rezirkulation von 20% auftritt, wird bei einem dem Dialysator 7 zugeführten Blutfluss von 200 ml eine Menge von 40 ml bereits wieder gereinigtem Blut und eine Menge von 160 ml von vollständig aufgeladenem, d.h. ungereinigtem Blut aus dem Patienten über den arteriellen Zugang 26 entnommen. Dies bedeutet, dass die Rückführung bzw. Rezirkulation eine nahezu 20% geringere Konzentration der Giftstoffe in arteriellem Zugang 26 verursacht, die dann zum Dialysator 7 fließen und durch diesen gereinigt werden.

Durch eine Differenzbildung der Absorbanz zwischen den beiden Messpunkten vor und nach der Erkennung einer plötzlichen Änderung der Rezirkulation bzw. nach der Erkennung einer Rezirkulation, kann die relative Änderung der Menge, z.B. an Giftstoffen, bestimmt werden. Hiervon kann bei plötzlichem Auftreten einer Rezirkulation oder Rezirkulationsänderung ausgegangen werden, da die Auswirkungen einer Rezirkulation auf die Clearance-Rate auf Grund des Konzentrationsgradienten vernachlässigbar oder allenfalls gering ist. Damit ist es je nach Stabilität des Signals möglich, durch die Änderung der Absorbanz die Menge der Rezirkulation zu bestimmen.

Unter der Annahme, dass sich die Konzentration von Blutparametern wie etwa von Giftstoffen im Blut des Patienten während der zwei Messpunkte vor und nach der Änderung, d.h. dem Auftreten einer Rezirkulation, nicht geändert hat, kann mit der folgenden Formel die Rezirkulation, bzw. die Größe der Rezirkulation, in folgender Weise bestimmt werden:

$$\Delta Rezirkulation = 1 - \left( \frac{\log_{10}\left( \frac{Detektor_0}{Detektorwert\_Soll\_i} \right)}{\log_{10}\left( \frac{Detektor_0}{Detektorwert\_i} \right)} \right) \qquad (3.8)$$

Der Grad der Rezirkulation, $\Delta$Rezirkulation, lässt sich hierbei durch Subtraktion des Ergebnisses einer Quotientenbildung aus den Zehnerlogarithmen der Quotienten des Detektor-Nullwert $Detektor_0$, jeweils bezogen auf den Detektorsollwert i bzw. den aktuellen Detektorwert i, vom Wert "1" ermitteln.

Bei Ausführungsbeispielen des erfindungsgemäßen Verfahrens und der erfindungsgemäßen Vorrichtung erfolgt die vorstehend beschriebene Messung vorzugsweise parallel zur Therapie und hat keinen Einfluss auf diese Dialysetherapie. Zudem ist die Messung ohne zusätzlichen technischen Aufwand möglich. Sofern Änderungen des Systems auftreten sollten, indem beispielsweise Flüsse, z.B. der Blutfluss, geändert werden und diese Änderungen Einfluss auf die Clearance haben, erfolgt vorzugsweise eine neue Berechnung der Steigung, d.h. der zu erwartenden Steigung des Detektorsignals und damit des Detektorsollwerts und dem gemäß der Grenzwerte.

Bei einem oder mehreren Ausführungsbeispielen der Erfindung kann zusätzlich auch noch der Druck beim Drucksensor 14 und/oder Drucksensor 8 überwacht werden. Diese ermöglicht dabei die Überwachung des Dialysators, um Fehlverhalten zu erkennen. Wenn erkannt wird, dass der mit den Drucksensoren 14, 8 erfasste Druck nicht parallel ansteigt oder die Differenz konstant bleibt, kann bei Auftreten einer plötzlichen Überschreitung oder auch Unterschreitung des jeweiligen Grenzwerts durch den aktuellen Detektorwert bzw. der Absorbanz auf eine Rezirkulation im Shunt geschlossen werden. Wenn demgegenüber erfasst wird, dass die Druckverläufe an den Drucksensoren 14, 8 nicht parallel ansteigen bzw. die Differenz wächst, indem das Signal an Sensor 14 ansteigt und keine Veränderung an Sensor 8 auftritt, kann ggf. eine Verklottung des Dialysators vorliegen, d.h. diese erkannt werden, die ebenfalls Einfluss auf die Clearance hat.

Die vorstehend beschriebene Beobachtung des Therapieverlaufs über die Auswertung der Detektorsignale des Sensors 12 dient dazu, eine plötzliche Rezirkulation zu detektieren und hierbei als Trigger entsprechende Maßnahmen und Messungen einzuleiten, um eine konstant hohe Dialyseeffektivität erhalten zu können.

Mit Ausführungsbeispielen der Erfindung kann somit während einer Therapie eine plötzliche Rezirkulation durch eine Änderung einer gemessenen Absorbanz im Abfluss des Dialysators kontinuierlich bzw. online, ohne zusätzliche Messungen detektiert werden. Dabei kann auch die Rezirkulationsgröße berechnet werden und bei einer plötzlichen Rezirkulation die geringere Dialyseeffektivität aufgezeigt werden.

Mit Ausführungsbeispielen der Erfindung ist eine frühzeitige Erkennung einer Veränderung des venösen und arteriellen Zugangs möglich. Eine Rezirkulation im Shunt wird als eine Fehlfunktion erkannt, sodass eine hierdurch bedingte Einschränkung der Effektivität der Dialyse ermittelt und ggf. entsprechende Gegenaktionen ergriffen werden können.

## Patentansprüche

1. Verfahren zum maschinen-seitigen Erfassen einer Rezirkulation von Blut oder einer Änderung einer Rezirkulation während des Betriebs einer Blutbehandlungsmaschine,

   wobei während des Betriebs aufeinanderfolgende Messwerte mittels eines Sensors (18) am Abfluss eines Dialysators (7) der Blutbehandlungsmaschine ermittelt werden, und bei dem für einen jeweiligen, vom Sensor (12) erzeugten Messwert ein Grenzwert vorgegeben wird, und bei dem dann, wenn der Messwert den Grenzwert erreicht oder überschreitet, eine Aktion wie etwa die Erzeugung eines optischen und/oder akustischen Warnhinweises eingeleitet wird,
   wobei ein von dem Sensor ermittelter aktueller Detektorwert in einen Absorbanzwert umgerechnet wird und ein Sollverlauf für die Absorbanz und ebenso für den Verlauf des Detektorsignals berechnet und überwacht wird, um eine plötzliche Rezirkulation bzw. eine plötzliche Rezirkulationsänderung aufgrund einer plötzlichen Veränderung der Absorbanz oder des Verlaufs des Detektorsignals zu erkennen;
   wobei zur Berechnung des Sollverlaufs der ermittelten Absorbanz und/oder des Sollverlaufs des Detektorsignals mindestens die nachfolgend angegebene Methode eingesetzt wird: der Sollverlauf des Detektorsignals oder der Absorbanz wird durch Linearisierung des Verlaufs des Detektorsignals berechnet, indem zu Beginn des Maschinenbetriebs oder ggf. auch nach einer Blutflussänderung die Steigung des Verlaufs der Detektorsignale ermittelt wird und diese Steigung als Sollwert für den weiteren Verlauf genutzt wird; und
   wobei die jeweiligen Grenzwerte für die jeweiligen Messwerte gleichartig wie der Sollverlauf berechnet werden, indem

   zu dem jeweiligen oder einem früheren oder einem erwarteten Messwert ein prozentualer oder fester Wert hinzuaddiert wird, und/oder
   der Grenzwert prozentual vom Sollwert zu jedem Zeitpunkt berechnet wird, und/oder
   der Grenzwert mittels eines Versatzwerts (Offset) bezogen auf den Sollverlauf zu jedem Zeitpunkt berechnet wird, und/oder
   mit Hilfe einer Prädiktion der Verlauf der theoretischen Messwerte ohne Rezirkulation berechnet wird und hierzu eine maximal zulässige obere Grenze als Grenzwert für die Abweichung ermittelt wird, und/oder
   auf der Basis des Sollverlaufs der Messwerte der Verlauf der Detektorsignale berechnet wird und auf dieser Basis eine maximal zulässige Abweichung für einen nachfolgenden Messwert als Grenzwert berechnet wird.

2. Verfahren nach Anspruch 1, bei dem der Grenzwert auf der Basis des aktuellen oder mindestens eines vorhergehenden Messwerts oder eines durch Prädiktion wie beispielsweise durch Extrapolation gewonnenen Vorhersagewerts berechnet wird.

3. Verfahren nach einem der vorangehenden Ansprüche, bei dem der Grenzwert als prozentualer oder fester Versatz (Offset) gegenüber dem aktuellen oder mindestens einem vorhergehenden Messwert oder dem durch Prädiktion gewonnenen Wert berechnet wird.

4. Verfahren nach einem der vorhergehenden Ansprüche, bei dem der Grenzwert auf der Basis einer erlaubten Rezirkulation berechnet wird.

5. Verfahren nach einem der vorhergehenden Ansprüche, bei dem der Sensor ein im Ultraviolettbereich (UV-Bereich) arbeitender Sensor ist und als Lichtquelle eine Leuchtdiode eingesetzt wird, die ein Signal mit einer Wellenlänge im Ultraviolettbereich, beispielsweise von ca. 280 nm, aussendet, und bei dem der Sensor einen oder vorzugsweise zwei Photodetektoren aufweist.

6. Verfahren nach einem der vorhergehenden Ansprüche, bei dem vor oder zu Beginns eines Behandlungs-Betriebs oder einer Messung, oder auch während eines Behandlungs-Betriebs oder einer Messung, eine Kalibrierung durchgeführt wird, bei der der Sensor eine definierte Flüssigkeit, vorzugsweise frische Dialysierflüssigkeit oder Wasser, misst und der hierbei gewonnene Messwert als Nullwert für die nachfolgende Auswertung herangezogen wird.

7. Verfahren nach einem der vorhergehenden Ansprüche, bei dem auf der Basis von mindestens zwei, vorzugsweise drei oder vier Messwerten Prädiktionswerte für erwartete zukünftige Messwerte berechnet werden und auf dieser Basis ein jeweils zugehöriger Grenzwert ermittelt wird.

8. Verfahren nach einem der vorhergehenden Ansprüche, bei dem der Druckabfall über den Blut-Einlass des Dialysators (7) bis zum Druck am Blut- Ausgang des Dialysators (7) mittels Drucksensoren (14, 8) überwacht wird, und dann, wenn die Differenz des Sensors (14) und des Sensors (8) den Grenzwert nicht erreicht oder nicht überschreitet, auf Rezirkulation geschlossen wird, während bei Überschreiten oder Erreichen des Grenzwerts auf Verklottung geschlossen wird.

9. Verfahren nach einem der vorhergehenden Ansprüche, bei dem die bei Erreichen oder Überschreiten des Grenzwerts vorgenommene Aktion in der Erzeugung eines akustischen und/oder optischen Warnsignals besteht.

10. Verfahren nach einem der vorhergehenden Ansprüche, bei dem nicht nur das Auftreten, sondern auch das Ausmaß einer Rezirkulation ermittelt wird, vorzugsweise durch Differenzbildung der Absorbanz zwischen zwei Messpunkten vor und nach der Erkennung einer Rezirkulation, um anhand der Änderung der Absorbanz die Menge bzw. Größe der Rezirkulation zu bestimmen.

11. Verfahren nach einem der vorangehenden Ansprüche, bei dem zur Berechnung des Sollverlaufs der ermittelten Absorbanz und/oder des Sollverlaufs des Detektorsignals ferner mindestens eine der nachfolgend angegebenen Methoden eingesetzt wird:

   der Sollverlauf wird durch Anpassung des exponentiellen Verlaufs der Absorbanz ermittelt, indem zu Beginn der des Maschinenbetriebs oder nach einer Blutflussänderung oder während eines Behandlungs- oder Maschinenbetriebs die Parameter des exponentiellen Verlaufs der Absorbanz ermittelt und als Sollwert für den weiteren Verlauf des Sollverlaufs genutzt werden, und/oder
   der Verlauf der Messwerte wird basierend auf einer Historie der Messwerte vorhergehender Maschinenbetriebe oder der letzten Steigungen der Messsignale hervorgehender Maschinenbetriebe ermittelt und hieraus wird der Sollverlauf berechnet, und/oder
   der Sollverlauf wird mit Hilfe der Systemparameter und der Patientendaten vorzugsweise in einem Modell berechnet.

12. Vorrichtung zum Erfassen einer Rezirkulation von Blut oder einer Änderung einer Rezirkulation während einer laufenden Blutbehandlung mittels eines Dialysators (7), mit einem am Abfluss des Dialysators (7) angeordneten Sensor (18) zur Ermittlung aufeinanderfolgender Messwerte während der Blutbehandlung, und einer Auswerteeinrichtung (19), die dazu ausgelegt ist, für einen jeweiligen, vom Sensor (18) erzeugten Messwert einen Grenzwert vorzugeben, und dann, wenn der Messwert den Grenzwert erreicht oder überschreitet, eine Aktion wie etwa die Erzeugung eines optischen und/oder akustischen Warnhinweises einzuleiten, umfassend

   Mittel zur Umrechnung eines von dem Sensor ermittelten aktuellen Detektorwerts in einen Absorbanzwert und zur Berechnung und Überwachung eines Sollverlaufs für die Absorbanz und ebenso für den Verlauf des Detektorsignals, um eine plötzliche Rezirkulation bzw. eine plötzliche Rezirkulationsänderung aufgrund einer plötzlichen Veränderung der Absorbanz oder des Verlaufs des Detektorsignals zu erkennen; und
Mittel zur Berechnung des Sollverlaufs der ermittelten Absorbanz und/oder des Sollverlaufs des Detektorsignals gemäß der nachfolgend angegebenen Methode: der Sollverlauf des Detektorsignals oder der Absorbanz wird durch Linearisierung des Verlaufs des Detektorsignals berechnet, indem zu Beginn des Maschinenbetriebs oder ggf. auch nach einer Blutflussänderung die Steigung des Verlaufs der Detektorsignale ermittelt wird und diese Steigung als Sollwert für den weiteren Verlauf genutzt wird.

13. Vorrichtung nach Anspruch 12, mit Mitteln zum maschinen-seitigen Erfassen der Rezirkulation von Blut oder der Änderung einer Rezirkulation während des Betriebs der Blutbehandlungsmaschine.

14. Vorrichtung nach Anspruch 12, mit Mitteln zum Berechnen des Grenzwerts auf der Basis des aktuellen oder mindestens eines vorhergehenden Messwerts oder eines durch Prädiktion wie beispielsweise durch Extrapolation gewonnenen Vorhersagewerts.

15. Vorrichtung nach einem der Ansprüche 12 bis 13, mit Mitteln zum Berechnen des Grenzwerts als prozentualer oder fester Versatz (Offset) gegenüber dem aktuellen oder mindestens einem vorhergehenden Messwert oder dem durch Prädiktion gewonnenen Wert.

16. Vorrichtung nach einem der vorhergehenden Ansprüche 12 bis 15, mit Mitteln zum Berechnen des Grenzwerts auf der Basis einer erlaubten Rezirkulation.

17. Vorrichtung nach einem der vorhergehenden Ansprüche 12 bis 16, bei der der Sensor ein im Ultraviolettbereich (UV-Bereich) arbeitender Sensor ist und als Lichtquelle eine Leuchtdiode eingesetzt ist, die ein Signal mit einer Wellenlänge im Ultraviolettbereich, beispielsweise von ca. 280 nm, aussendet, und bei der der Sensor einen oder vorzugsweise zwei Photodetektoren aufweist.

18. Vorrichtung nach einem der vorhergehenden Ansprüche 12 bis 17, mit Mitteln zur Durchführung einer Kalibrierung vor oder zu Beginns eines Behandlungs-Betriebs oder einer Messung, oder auch während eines Behandlungs-Betriebs oder einer Messung, wobei der Sensor bei der Kalibrierung eine definierte Flüssigkeit, vorzugsweise frische Dialysierflüssigkeit oder Wasser, misst und der hierbei gewonnene Messwert als Nullwert für die nachfolgende Auswertung herangezogen wird.

19. Vorrichtung nach einem der vorhergehenden Ansprüche 12 bis 18, mit Mitteln zur Berechnung der jeweiligen Grenzwerte für die jeweiligen Messwerte gleichartig wie der Sollverlauf, indem zu dem jeweiligen oder einem früheren oder einem erwarteten Messwert ein prozentualer oder fester Wert hinzuaddiert wird, und/oder

   zur Berechnung des Grenzwerts prozentual vom Sollwert zu jedem Zeitpunkt, und/oder
   zur Berechnung des Grenzwerts mittels eines Versatzwerts (Offset) bezogen auf den Sollverlauf zu jedem Zeitpunkt, und/oder
   zur Berechnung, mit Hilfe einer Prädiktion, des Verlaufs der theoretischen Messwerte ohne Rezirkulation und Ermittlung hierzu einer maximal zulässigen oberen Grenze als Grenzwert für die Abweichung, und/oder
   zur Berechnung, auf der Basis des Sollverlaufs der Messwerte, des Verlaufs der Detektorsignale und auf dieser Basis einer maximal zulässigen Abweichung für einen nachfolgenden Messwert als Grenzwert.

20. Vorrichtung nach einem der vorhergehenden Ansprüche 12 bis 19, mit Mitteln zur Berechnung, auf der Basis von mindestens zwei, vorzugsweise drei oder vier Messwerten, von Prädiktionswerten für erwartete zukünftige Messwerte und Ermittlung eines jeweils zugehörigen Grenzwerts auf dieser Basis.

21. Vorrichtung nach einem der vorhergehenden Ansprüche 12 bis 20, mit Drucksensoren (14, 8) zur Überwachung des Druckabfalls über den Blut-Einlass des Dialysators (7) bis zum Druck am Blut-Ausgang des Dialysators (7), und mit Mitteln zum Schließen dann, wenn die Differenz des Sensors (14) und Sensors (8) den Grenzwert nicht erreicht oder nicht überschreitet, auf Rezirkulation, während bei Überschreiten oder Erreichen des Grenzwerts auf Verklottung geschlossen wird.

22. Vorrichtung nach einem der vorhergehenden Ansprüche 12 bis 21, bei der die bei Erreichen oder Überschreiten des Grenzwerts vorgenommene Aktion in der Erzeugung eines akustischen und/oder optischen Warnsignals und/oder in der Veränderung oder Abschaltung der Blutströmungsrate des für die Dialyse zu entnehmenden Patientenbluts besteht.

23. Vorrichtung nach einem der vorhergehenden Ansprüche 12 bis 22, mit Mitteln zur Ermittlung nicht nur des Auftretens, sondern auch des Ausmaßes einer Rezirkulation, vorzugsweise durch Differenzbildung der Absorbanz zwischen zwei Messpunkten vor und nach der Erkennung einer Rezirkulation, um anhand der Änderung der Absorbanz die Menge bzw. Größe der Rezirkulation zu bestimmen.

24. Vorrichtung nach einem der vorhergehenden Ansprüche 12 bis 23, ferner umfassend
Mittel zur Berechnung des Sollverlaufs der ermittelten Absorbanz und/oder des Sollverlaufs des Detektorsignals gemäß ferner mindestens einer der nachfolgend angegebenen Methoden:

> der Sollverlauf wird durch Anpassung des exponentiellen Verlaufs der Absorbanz ermittelt, indem zu Beginn der des Maschinenbetriebs oder nach einer Blutflussänderung oder während eines Behandlungs- oder Maschinenbetriebs die Parameter des exponentiellen Verlaufs der Absorbanz ermittelt und als Sollwert für den weiteren Verlauf des Sollverlaufs genutzt werden, und/oder
> der Verlauf der Messwerte wird basierend auf einer Historie der Messwerte vorhergehender Maschinenbetriebe oder der letzten Steigungen der Messsignale hervorgehender Maschinenbetriebe ermittelt und hieraus wird der Sollverlauf berechnet, und/oder
> der Sollverlauf wird mit Hilfe der Systemparameter und der Patientendaten vorzugsweise in einem Modell berechnet.

**Claims**

1. A method for the machine-side detection of a recirculation of blood or a change of a recirculation during the operation of a blood treatment machine,

> wherein during the operation, successive measurement values are determined via a sensor (18) at the drain of a dialyzer (7) of the blood treatment machine, and wherein a threshold value is predetermined for a respective measurement value generated by the sensor (12), and wherein when the measurement value reaches or exceeds the threshold value, an action such as the generation of an optical and/or acoustic warning notice is initiated, wherein a current detector value determined by the sensor is converted into an absorbance value and a target course for the absorbance and also for the course of the detector signal is calculated and monitored in order to detect a sudden recirculation or a sudden change in recirculation due to a sudden change in the absorbance or the course of the detector signal;
> wherein at least the following procedure is used to calculate the target course of the determined absorbance and/or of the target course of the detector signal: the target course of the detector signal or of the absorbance is calculated by linearization of the course of the detector signal, in that the slope of the course of the detector signal is determined at the beginning of the machine operation or, if applicable, also after a change in blood flow, and this slope is used as a target value for the further course; and
> wherein the respective threshold values for the respective measurement values are calculated in the same manner as the target course, by
>
>> adding a percentage or fixed value to the respective or a previous or an expected measurement value, and/or the threshold value is calculated as a percentage of the target value at each point in time, and/or the threshold value is calculated via an offset value (offset) relative to the target course at each time, and/or the course of the theoretical measurement values without recirculation is calculated via a prediction and for this purpose a maximum permissible upper limit is determined as threshold value for the deviation, and/or the course of the detector signals is calculated on the basis of the target course of the measurement values, and on this basis a maximum permissible deviation for a successive measurement value is calculated as a threshold value.

2. The method of claim 1, wherein the threshold value is calculated based on the current or at least one previous measurement value or on a prediction value obtained by prediction such as extrapolation.

3. The method according to one of the preceding claims, wherein the threshold value is calculated as a percentage or fixed offset from the current or at least one previous measurement value or from the value obtained by prediction.

4. The method according to one of the preceding claims, wherein the threshold value is calculated on the basis of an allowed recirculation.

5. The method according to one of the preceding claims, wherein the sensor is a sensor operating in the ultraviolet range (UV range) and a light emitting diode is used as the light source, which emits a signal with a wavelength in the ultraviolet range, for example of about 280 nm, and wherein the sensor comprises one or preferably two photodetectors.

6. The method according to one of the preceding claims, wherein before or at the beginning of a treatment operation or a measurement, or also during a treatment operation or a measurement, a calibration is carried out in which the sensor measures a defined liquid, preferably fresh dialysis liquid or water, and the measurement value obtained in this process is used as zero value for the subsequent evaluation.

7. The method according to one of the preceding claims, wherein prediction values for expected future measurement values are calculated on the basis of at least two, preferably three or four measurement values, and a respective associated threshold value is determined on this basis.

8. The method according to one of the preceding claims, wherein the pressure drop across the blood inlet of the dialyzer (7) to the pressure at the blood outlet of the dialyzer (7) is monitored via pressure sensors (14, 8), and if the difference of the sensor (14) and the sensor (8) does not reach or exceed the threshold value, recirculation is concluded, whereas if the threshold value is exceeded or reached, clotting is concluded.

9. The method according to one of the preceding claims, wherein the action taken when the threshold value is reached or exceeded consists in generating an acoustic and/or optical warning signal.

10. The method according to one of the preceding claims, wherein not only the occurrence but also the extent of a recirculation is determined, preferably by difference formation of the absorbance between two measuring points before and after the detection of a recirculation, in order to determine the amount or magnitude of the recirculation on the basis of the change in absorbance.

11. The method according to one of the preceding claims, wherein for calculating the target course of the determined absorbance and/or of the target course of the detector signal, at least one of the following procedures is furthermore used:

the target course is determined by adjusting the exponential course of the absorbance by determining the parameters of the exponential course of the absorbance at the beginning of the machine operation or after a blood flow change or during a treatment or machine operation and using them as a target value for the further course of the target course, and/or
the course of the measurement values is determined based on a history of the measurement values of preceding machine operations or the last slopes of the measurement signals of preceding machine operations and from this the target course is calculated, and/or
the target course is calculated with the aid of the system parameters and the patient data, preferably in a model.

12. A device for detecting a recirculation of blood or a change in a recirculation during an ongoing blood treatment via a dialyzer (7), having a sensor (18) arranged at the drain of the dialyzer (7) for determining successive measurement values during the blood treatment, and an evaluation device (19) which is configured to preset a threshold value for a respective measurement value generated by the sensor (18) and, when the measurement value reaches or exceeds the threshold value, to initiate an action such as the generation of an optical and/or acoustic warning notice, comprising

means for converting a current detector value determined by the sensor into an absorbance value and for calculating and monitoring a target course for the absorbance and also for the course of the detector signal to detect a sudden recirculation or change in recirculation due to a sudden change in the absorbance or the course of the detector signal; and
means for calculating the target course of the determined absorbance and/or of the target course of the detector signal according to the following procedure: the target course of the detector signal or the absorbance is calculated by linearization of the course of the detector signal by determining the slope of the course of the detector signal at the beginning of the machine operation or, if applicable, also after a change in blood flow and using this slope as the target value for the further course.

13. The device according to claim 12, comprising means for machine-side detection of the recirculation of blood or the change of a recirculation during the operation of the blood treatment machine.

14. The device according to claim 12, comprising means for calculating the threshold value based on the current or at least one previous measurement value or a prediction value obtained via prediction such as extrapolation.

15. The device according to one of claims 12 to 13, comprising means for calculating the threshold value as a percentage or fixed offset from the current or at least one previous measurement value or the value obtained by prediction.

16. The device according to one of claims 12 to 15, comprising means for calculating the threshold value based on an allowed recirculation.

17. The device according to one of the preceding claims 12 to 16, wherein the sensor is a sensor operating in the ultraviolet range (UV range) and a light emitting diode is used as the light source, which emits a signal having a wavelength in the ultraviolet range, for example of about 280 nm, and wherein the sensor comprises one or preferably two photodetectors.

18. The device according to one of the preceding claims 12 to 17, with means for carrying out a calibration before or at the beginning of a treatment operation or a measurement, or also during a treatment operation or a measurement, wherein the sensor measures a defined liquid, preferably fresh dialysis liquid or water, during the calibration and the measurement value obtained in this process is used as zero value for the subsequent evaluation.

19. The device according to one of the preceding claims 12 to 18, comprising means for calculating the respective threshold values for the respective measurement values of the same kind as the target course by adding a percentage or fixed value to the respective or a previous or an expected measurement value, and/or

    for calculating the threshold value as a percentage of the target value at any time, and/or
    for calculating the threshold value via an offset value (offset) relative to the target course at any time, and/or
    for calculating, via a prediction, the course of the theoretical measurement values without recirculation and determining a maximum permissible upper limit as threshold value for the deviation, and/or
    for calculating, on the basis of the target course of the measurement values, the course of the detector signals and on this basis a maximum permissible deviation for a subsequent measurement value as threshold value.

20. The device according to one of claims 12 to 19, comprising means for calculating, on the basis of at least two, preferably three or four measurement values, prediction values for expected future measurement values and determining a respective associated threshold value on this basis.

21. The device according to one of the preceding claims 12 to 21, comprising pressure sensors (14, 8) for monitoring the pressure drop across the blood inlet of the dialyzer (7) to the pressure at the blood outlet of the dialyzer (7), and means for concluding recirculation when the difference of the sensor (14) and sensor (8) does not reach or exceed the threshold value, while concluding clotting when exceeding or reaching the threshold value.

22. The device according to one of claims 12 to 21, wherein the action taken upon reaching or exceeding the threshold value consists of generating an acoustic and/or visual warning signal and/or changing or shutting off the blood flow rate of the patient's blood to be removed for dialysis.

23. The device according to one of the preceding claims 12 to 22, comprising means for determining not only the occurrence but also the extent of recirculation, preferably by difference formation of the absorbance between two measuring points before and after detection of recirculation, in order to determine the amount or magnitude of recirculation on the basis of the change in absorbance.

24. The device according to one of claims 12 to 23, furthermore comprising
    means for calculating the target course of the determined absorbance and/or of the target course of the detector signal according to at least one of the following procedures:

    the target course is determined by adjusting the exponential course of the absorbance by determining the parameters of the exponential course of the absorbance at the beginning of the machine operation or after a blood flow change or during a treatment or machine operation and using them as a target value for the further course of the target course, and/or

the course of the measurement values is determined based on a history of the measurement values of preceding machine operations or the last slopes of the measurement signals of preceding machine operations and from this the target course is calculated, and/or

the target course is calculated with the aid of the system parameters and the patient data, preferably in a model.

## Revendications

1. Procédé destiné à saisir du côté machine une recirculation de sang ou une modification d'une recirculation pendant le fonctionnement d'une machine de traitement du sang,

dans lequel pendant le fonctionnement des valeurs mesurées qui se succèdent sont établies au moyen d'un capteur (18) au niveau du drain d'un dialyseur (7) de la machine de traitement du sang, et où une valeur limite est prédéfinie pour une valeur mesurée respective produite par le capteur (12), et où une action comme par exemple la production d'un message d'alerte optique et/ou acoustique est déclenchée dès lors que la valeur mesurée a atteint ou dépassé la valeur limite,

dans lequel une valeur de détecteur en cours établie par le capteur est convertie en une valeur d'absorbance et une évolution nominale pour l'absorbance et également pour l'évolution du signal de détecteur est calculée et surveillée pour détecter une recirculation soudaine ou une modification de recirculation soudaine du fait d'une modification soudaine de l'absorbance ou de l'évolution du signal de détecteur ;

dans lequel pour calculer l'évolution nominale de l'absorbance établie et/ou de l'évolution nominale du signal de détecteur, au moins la méthode fournie ci-après est utilisée :

l'évolution nominale du signal de détecteur ou de l'absorbance est calculée par linéarisation de l'évolution du signal de détecteur, où au début du fonctionnement de machine ou le cas échéant également après une modification de flux sanguin, la pente de l'évolution du signal de détecteur est établie et cette pente est employée en tant que valeur nominale pour l'évolution suivante ; et

dans lequel les valeurs limites respectives pour les valeurs mesurées respectives sont calculées de façon similaire à l'évolution nominale, où

une valeur en pourcentage ou fixe est ajoutée à la valeur mesurée respective ou à une valeur mesurée antérieure ou attendue, et/ou

la valeur limite est calculée en pourcentage de la valeur nominale à chaque point dans le temps, et/ou la valeur limite est calculée au moyen d'une valeur de décalage (offset) par rapport à l'évolution nominale à chaque point dans le temps, et/ou

l'évolution des valeurs mesurées théoriques sans recirculation est calculée à l'aide d'une prédiction et à cet égard une limite supérieure maximale autorisée est établie pour l'écart en tant que valeur limite, et/ou l'évolution des signaux de détecteur est calculée sur la base de l'évolution nominale des valeurs mesurées, et sur cette base un écart maximal autorisé est calculé pour une valeur mesurée suivante en tant que valeur limite.

2. Procédé selon la revendication 1, dans lequel la valeur limite est calculée sur la base de la valeur mesurée en cours ou au moins d'une valeur mesurée précédente ou d'une valeur prédictive obtenue par prédiction comme par exemple par extrapolation.

3. Procédé selon l'une des revendications précédentes, dans lequel la valeur limite est calculée en tant que décalage (offset) en pourcentage ou fixe par rapport à la valeur mesurée en cours ou au moins à une valeur mesurée précédente ou à la valeur obtenue par prédiction.

4. Procédé selon l'une des revendications précédentes, dans lequel la valeur limite est calculée sur la base d'une recirculation autorisée.

5. Procédé selon l'une des revendications précédentes, dans lequel le capteur est un capteur qui travaille dans la plage ultraviolette (plage UV) et en tant que source lumineuse est utilisée une diode électroluminescente qui émet un signal avec une longueur d'onde dans la plage ultraviolette, par exemple de 280 nm environ, et dans lequel le capteur présente un ou de préférence deux photodétecteurs.

6. Procédé selon l'une des revendications précédentes, dans lequel avant ou au début d'un fonctionnement de traitement ou d'une mesure, ou également pendant un fonctionnement de traitement ou une mesure, un étalonnage est effectué, dans lequel le capteur mesure un fluide défini, de préférence un fluide de dialyse frais ou de l'eau, et la valeur mesurée ainsi obtenue est utilisée en tant que valeur zéro pour l'évaluation suivante.

7. Procédé selon l'une des revendications précédentes, dans lequel des valeurs de prédiction pour des valeurs mesurées attendues à l'avenir sont calculées sur la base d'au moins deux, de préférence trois ou quatre valeurs mesurées, et une valeur limite respectivement correspondante est établie sur cette base.

8. Procédé selon l'une des revendications précédentes, dans lequel la chute de pression est surveillée au moyen de capteurs de pression (14, 8) via l'admission de sang du dialyseur (7) et jusqu'à la pression au niveau de la sortie de sang du dialyseur (7), et il est conclu à une recirculation dès lors que la différence entre le capteur (14) et le capteur (8) n'a pas atteint ou n'a pas dépassé la valeur limite, alors qu'il est conclu à un engorgement en cas de dépassement ou d'atteinte de la valeur limite.

9. Procédé selon l'une des revendications précédentes, dans lequel l'action entreprise en cas d'atteinte ou de dépassement de la valeur limite consiste à produire un signal d'alerte acoustique et/ou optique.

10. Procédé selon l'une des revendications précédentes, dans lequel est établie non seulement l'apparition mais aussi l'ampleur d'une recirculation, de préférence par formation de différence de l'absorbance entre deux points mesurés avant et après la détection d'une recirculation pour déterminer le volume ou la taille de la recirculation en se fondant sur la modification de l'absorbance.

11. Procédé selon l'une des revendications précédentes, dans lequel pour calculer l'évolution nominale de l'absorbance établie et/ou de l'évolution nominale du signal de détecteur, au moins l'une des méthodes fournies ci-après est en outre utilisée :

l'évolution nominale est établie par rajustement de l'évolution exponentielle de l'absorbance, où au début du fonctionnement de machine ou après une modification de flux sanguin ou pendant un fonctionnement de traitement ou de machine, les paramètres de l'évolution exponentielle de l'absorbance sont établis et employés en tant que valeur nominale pour l'évolution suivante de l'évolution nominale, et/ou
l'évolution des valeurs mesurées est établie sur la base d'un historique des valeurs mesurées de fonctionnements de machine précédents ou des dernières pentes des signaux mesurés de fonctionnements de machine précédents, et partant de là est calculée l'évolution nominale, et/ou
l'évolution nominale est calculée de préférence dans un modèle à l'aide des paramètres de système et des données de patient.

12. Dispositif destiné à saisir une recirculation de sang ou une modification d'une recirculation pendant un traitement du sang en cours au moyen d'un dialyseur (7), avec un capteur (18) agencé au niveau du drain d'un dialyseur (7) pour établir des valeurs mesurées qui se succèdent pendant le traitement du sang, et un équipement d'évaluation (19) qui est conçu pour prédéfinir une valeur limite pour une valeur mesurée respective produite par le capteur (18), et déclencher une action comme par exemple la production d'un message d'alerte optique et/ou acoustique dès lors que la valeur mesurée a atteint ou dépassé la valeur limite, qui comprend

des moyens pour convertir une valeur de détecteur en cours établie par le capteur en une valeur d'absorbance et pour calculer et surveiller une évolution nominale pour l'absorbance et également pour l'évolution du signal de détecteur afin de détecter une recirculation soudaine ou une modification de recirculation soudaine du fait d'une modification soudaine de l'absorbance ou de l'évolution du signal de détecteur ; et
des moyens pour calculer l'évolution nominale de l'absorbance établie et/ou de l'évolution nominale du signal de détecteur selon la méthode fournie ci-après : l'évolution nominale du signal de détecteur ou de l'absorbance est calculée par linéarisation de l'évolution du signal de détecteur, où au début du fonctionnement de machine ou le cas échéant également après une modification de flux sanguin, la pente de l'évolution du signal de détecteur est établie et cette pente est employée en tant que valeur nominale pour l'évolution suivante.

13. Dispositif selon la revendication 12, avec des moyens pour saisir du côté machine la recirculation de sang ou la modification d'une recirculation pendant le fonctionnement de la machine de traitement du sang.

14. Dispositif selon la revendication 12, avec des moyens pour calculer la valeur limite sur la base de la valeur mesurée en cours ou au moins d'une valeur mesurée précédente ou d'une valeur prédictive obtenue par prédiction comme par exemple par extrapolation.

**15.** Dispositif selon l'une des revendications 12 à 13, avec des moyens pour calculer la valeur limite en tant que décalage (offset) en pourcentage ou fixe par rapport à la valeur mesurée en cours ou au moins à une valeur mesurée précédente ou à la valeur obtenue par prédiction.

**16.** Dispositif selon l'une des revendications précédentes 12 à 15, avec des moyens pour calculer la valeur limite sur la base d'une recirculation autorisée.

**17.** Dispositif selon l'une des revendications précédentes 12 à 16, dans lequel le capteur est un capteur qui travaille dans la plage ultraviolette (plage UV) et en tant que source lumineuse est utilisée une diode électroluminescente qui émet un signal avec une longueur d'onde dans la plage ultraviolette, par exemple de 280 nm environ, et dans lequel le capteur présente un ou de préférence deux photodétecteurs.

**18.** Dispositif selon l'une des revendications précédentes 12 à 17, avec des moyens pour effectuer un étalonnage avant ou au début d'un fonctionnement de traitement ou d'une mesure, ou également pendant un fonctionnement de traitement ou une mesure, dans lequel lors de l'étalonnage le capteur mesure un fluide défini, de préférence un fluide de dialyse frais ou de l'eau, et la valeur mesurée ainsi obtenue est utilisée en tant que valeur zéro pour l'évaluation suivante.

**19.** Dispositif selon l'une des revendications précédentes 12 à 18, avec des moyens

pour calculer les valeurs limites respectives pour les valeurs mesurées respectives de façon similaire à l'évolution nominale, où une valeur en pourcentage ou fixe est ajoutée à la valeur mesurée respective ou à une valeur mesurée antérieure ou attendue, et/ou
pour calculer la valeur limite en pourcentage de la valeur nominale à chaque point dans le temps, et/ou
pour calculer la valeur limite au moyen d'une valeur de décalage (offset) par rapport à l'évolution nominale à chaque point dans le temps, et/ou
pour calculer à l'aide d'une prédiction l'évolution des valeurs mesurées théoriques sans recirculation et à cet égard une limite supérieure maximale autorisée est établie en tant que valeur limite pour l'écart, et/ou
pour calculer l'évolution des signaux de détecteur sur la base de l'évolution nominale des valeurs mesurées, et sur cette base un écart maximal autorisé pour une valeur mesurée suivante en tant que valeur limite.

**20.** Dispositif selon l'une des revendications précédentes 12 à 19, avec des moyens pour calculer des valeurs de prédiction pour des valeurs mesurées attendues à l'avenir sur la base d'au moins deux, de préférence trois ou quatre valeurs mesurées, et établir un valeur limite respectivement correspondante sur cette base.

**21.** Dispositif selon l'une des revendications précédentes 12 à 20, avec des capteurs de pression (14, 8) pour surveiller la chute de pression via l'admission de sang du dialyseur (7) et jusqu'à la pression au niveau de la sortie de sang du dialyseur (7), et avec des moyens pour conclure à une recirculation dès lors que la différence entre le capteur (14) et le capteur (8) n'a pas atteint ou n'a pas dépassé la valeur limite, alors qu'il est conclu à un engorgement en cas de dépassement ou d'atteinte de la valeur limite.

**22.** Dispositif selon l'une des revendications précédentes 12 à 21, dans lequel l'action entreprise en cas d'atteinte ou de dépassement de la valeur limite consiste à produire un signal d'alerte acoustique et/ou optique et/ou à modifier ou arrêter le débit de sang du sang de patient à prélever pour la dialyse.

**23.** Dispositif selon l'une des revendications précédentes 12 à 22, avec des moyens pour établir non seulement l'apparition mais aussi l'ampleur d'une recirculation, de préférence par formation de différence de l'absorbance entre deux points mesurés avant et après la détection d'une recirculation afin de déterminer le volume ou la taille de la recirculation en se fondant sur la modification de l'absorbance.

**24.** Dispositif selon l'une des revendications précédentes 12 à 23, qui comprend en outre
des moyens pour calculer l'évolution nominale de l'absorbance établie et/ou de l'évolution nominale du signal de détecteur selon en outre au moins l'une des méthodes fournies ci-après:

l'évolution nominale est établie par rajustement de l'évolution exponentielle de l'absorbance, où au début du fonctionnement de machine ou après une modification de flux sanguin ou pendant un fonctionnement de traitement ou de machine, les paramètres de l'évolution exponentielle de l'absorbance sont établis et employés en tant que valeur nominale pour l'évolution suivante de l'évolution nominale, et/ou

l'évolution des valeurs mesurées est établie sur la base d'un historique des valeurs mesurées de fonctionnements de machine précédents ou des dernières pentes des signaux mesurés de fonctionnements de machine précédents, et partant de là est calculée l'évolution nominale, et/ou

l'évolution nominale est calculée de préférence dans un modèle à l'aide des paramètres de système et des données de patient.

Fig. 1

Fig. 2

Fig. 3

Fig. 4

Fig. 5

**IN DER BESCHREIBUNG AUFGEFÜHRTE DOKUMENTE**

**In der Beschreibung aufgeführte Patentdokumente**

- DE 69916053 T2
- EP 2292283 A1
- US 2007083145 A1
- US 2008097272 A1
- US 2009054822 A1
- WO 2014044365 A1